# EUROPEAN PATENT SPECIFICATION

(11) **EP 0 629 240 B1**
(45) Date of publication and mention of the grant of the patent: **08.05.2002**
(21) Application number: 93904235.4
(22) Date of filing: 19.02.1993
(51) Int. Cl.: C12N 15/13, C07K 16/00, A61K 39/395

(54) **ALTERED ANTIBODIES, PRODUCTS AND PROCESSES RELATING THERETO**
Veränderte Antikörper, damit zusammenhängende Produkte und Verfahren
ANTICORPS MODIFIES, PRODUITS ET PROCEDES S'Y RAPPORTANT

(30) Priority: 19.02.1992 GB 9203459
(43) Date of publication of application: 21.12.1994
(73) Proprietor: SB2 Inc., Danville, California 94526 (US)
(72) Inventor: WINTER, Gregory, Paul, Cambridge CB1 4UT (GB); CARR, Francis Joseph, Balmedie, Aberdeenshire AB23 8XU (GB); HARRIS, William Joseph, Carnoustie, Angus DD7 6DR (GB)
(74) Representative: O'Brien, Caroline Jane
(86) International application number: GB9300363
(87) International publication number: WO9317105

(56) References cited:
- WO-A-90/07861
- SCIENCE. vol. 238, 20 November 1987, LANCASTER, PA US pages 1088 - 1094 K. RAJEWSKI ET AL. 'Evolutionary and somatic selection of the antibody repertoire in the mouse'
- MOLECULAR IMMUNOLOGY vol. 28, no. 4/5, May 1991, pages 489 - 498 E.A. PADLAN 'A possible procedure for reducing the immunogenecity of antibody variable domains while preserving their ligand-binding properties' cited in the application
- BIOTECHNOLOGY vol. 9, no. 3, March 1991, NEW YORK US pages 266 - 271 P.R.TEMPEST ET AL. 'Reshaping a human monoclonal antibody to inhibit human respiratory syncytial virus infection in vivo' cited in the application

## Description

The present invention concerns altered antibodies and products and processes relating to such antibodies.

Processes are described for the conversion of a given antibody sequence by recombinant DNA techniques towards the sequence of a known germ-line equivalent of the same or a different species.

The application of recombinant DNA technology to monoclonal antibodies has enabled man-made improvements in these antibodies to be implemented. In particular, recombinant antibodies with improved application in human healthcare have been created. The hybridoma technology of Köhler and Milstein (Köhler, G. and Milstein, C., Nature, 256, 495-497, 1975) has been used for the creation of many thousands of rodent monoclonal antibodies but it has proved very difficult to use this technology to create human antibodies.

EP125023 (Genentech) and EP120694 (Celltech) disclose the development of chimaeric antibodies through recombinant DNA comprising antibody variable (v) and constant (C) regions from different species and sources. Typically, this technology has been applied to creation of pharmaceutical antibodies with mouse V regions and human constant regions such as those described by LoBuglio, A.F. et al, Proc. Natl. Acad. Sci. USA, 86, 4220-4224, 1989. Nevertheless, the foreign V region in such antibodies is likely to provoke an immune response (see Bruggemann, M. et al., J. Exp. Med., 170, 2153-2157, 1989) thus limiting therapeutic effectiveness.

An alternative to chimaeric antibodies is taught by GB2188638 (Winter) whereby the antibody V region comprises of complementarity determining regions (CDRs) and V region frameworks from different sources. The process for producing such re-shaped (CDR-grafted) antibodies has been applied to the creation of pharmaceutical antibodies incorporating rodent CDRs, human V region frameworks and human constant regions (Riechmann, L. et al., Nature, 332, 323-327, 1988). Thus the V region in such antibodies is less likely to provoke an immune response in human patients than the corresponding region in the previously described chimaeric antibodies.

The ontogeny of antibody production in living mammals is a complex process, but nevertheless has been well described for example in Roitt, I., Essential Immunology, Blackwell Scientific Publishers. All mammals have genetic loci corresponding to immunoglobulin heavy and light chains which comprise multiple segments of V region genes. For example, there are about 500 mouse immunoglobulin heavy chain genes in addition to multiple constant region genes, several joining ("J") and diversity ("D") segments (the latter utilized in heavy chains only).

The initial germ-line configuration is preserved in most cells with the major exception of B cells where, as the cell matures, the immunoglobulin heavy and light chain V regions are subjected to recombination events whereby a specific V region gene becomes juxtaposed to D (heavy chain only), J and constant regions. The very high number of permutations of segments and domains coupled with some "slippage" in the exact points of association (recombination) give rise to a great variety of primary antibody molecules which may be displayed by immature B cells. Nevertheless, these antibody molecules include V regions whose amino acid sequences correspond to those encoded by germ-line V region genes.

Such germ-line V region antibody molecules are presumed to have been presented to the immune system early in the life of the organism and are thus recognised as "self".

Following subsequent exposures of antibody-bearing immature B cells to antigen, both heavy and light chain immunoglobulin V region genes are subject to somatic mutation events from which mature B cells producing higher affinity antibodies derive. Thus their sequences may begin to diversify away from the initial germ-line configuration.

This invention is based partly on the knowledge that during somatic mutation of V region genes in the maturation of B cells, not only do the CDRs mutate, but also the V region frameworks (in which the majority of residues are thought not to engage in, or influence antigen binding to any significant degree). The consequence of such apparently random somatic mutation in V region frameworks is the creation of antibody molecules with "non germ-line" frameworks including amino acid sequences which may be recognised as "foreign" instead of "self" by the organism.

In addition, the invention is also based partly upon the finding that whilst individual CDRs (defined on the basis of their hypervariability between different antibodies) are extensively subjected to somatic mutation, individual antibody molecules bind to antigens using only part of the total CDR amino acid sequences. Thus parts of the CDR sequences may be redundant with respect to antigen binding and random mutation may produce within such regions, amino acid sequences which have no deleterious effect on antigen binding but may be recognized as "foreign" entities.

Thus based on this knowledge, the present invention discloses altered antibodies which comprise one or more selected germ-line amino acid residues which replace one or more corresponding somatically mutated residues in a native antibody. These antibodies may have V regions comprising CDRs which provide the antibody with capacity to bind a specific antigen and predominantly germ-line framework sequences. The present invention also provides processes for making a gene for use in preparing such antibodies. A gene may encode a heavy chain and/or a light chain. Optionally the CDRs may have residues corresponding to germ-line sequences. The genes may be used in expression processes for making antibodies and hence provide for the conversion of antibodies of one species to germ-line equivalents of the same or another species and, the conversion of recombinant (for example re-shaped) antibodies into germ-line equivalents.

Altered antibodies with selected germ-line amino acid residues replacing one or more corresponding somatically mutated residues in a native antibody, and more particularly altered antibodies with V region selected and predominantly germ-line frameworks and optionally, germ-line CDR residues together with processes for producing such antibodies, have not previously been described in the scientific or patent literature.

Rajewsky et *al. (Science* 238:1088-1094 (1987)) describe the evolutionary selection of the variable regions of the antibodies. This selection affects both the diversity of V region genes in the germline and their expression in the B lymphocyte population and its subsets. In a defined phase of B cell differentiation, new sets of V regions are generated from the existing repertoire through somatic hypermutation.

In the recombinant chimaeric antibodies (for example, described in Rudikoff, S. et al., Proc. Natl. Acad. Sci. USA, 79, 1979-80, 1982; Bruggemann, M. et al., EMBO J., 1, 629-634, 1982; Oi, V.T. et al., Proc. Natl. Acad. Sci. USA, 80, 825-829, 1983; Neuberger M.S. et al., EMBO J., 2, 1373-1378, 1983; Boulianne, G.L. et al., Nature, 312, 643-646, 1984; EP125023 (Genentech); Neuberger M. et al., Nature 314, 268-270, 1985) the V region gene fragments were derived from hybridoma cells producing mature high-affinity monoclonal antibodies. Thus immature antibodies with closer to germ-line V regions were not envisaged.

Similarly in the recombinant reshaped (CDR-grafted) antibodies (for example, humanised antibodies described in Jones, P.T. et al., Nature, 321, 522-525, 1986; Riechmann, L. et al., 1988, loc. cit.; Verhoeyen, M. et al., Science, 239, 1534-1536, 1988; Queen, C. et al., Proc. Natl. Acad. Sci. USA, 86, 10029-10033, 1989; Tempest, P. et al., Biotechnology, 9, 266-271, 1991; Gorman, S.D. et al., Proc. Natl. Acad. Sci. USA, 88, 4181-4185, 1991; Co., M.S. et al., Proc. Natl. Acad. Sci. USA, 88, 2869-2873, 1991, J. Adair et al., PCT/GB90/02017), the CDRs were derived from murine hybridoma cells producing mature high affinity monoclonal antibodies, whilst human V region frameworks were derived from mature antibodies or myeloma proteins.

In none of these descriptions of chimaeric or reshaped antibodies has the use of germ-line V region frameworks been envisaged.

Padlan et al., (Mol. Immunology 28 No.4/5 p489-498 (1991)), describe a "re-surfacing" (or "veneering") technique which is based on the premise that since the antigenicity of a molecule is dependant on its surface, determining and replacing the exposed residues of an allogenic antibody with those usually found in host antibodies would reduce the problem of recognition and thus the problem of antigenicity. This possibility has been explored with respect to the humanisation of mouse antibodies.

However, the teaching is of replacing the exposed residues with residues of the other species which are again characteristic of an antibody which has undergone somatic mutation during B cell maturation. The teaching did not extend to the use of germ-line V region framework sequences. Furthermore, the approach described by Padlan et al., involves using only partial sequences, namely only the exposed surface residues. No disclosure was made in relation to the use of germ-line amino acid residues for example, V region frameworks as disclosed herein.

Thus the disclosures of the present application now provide for the "re-surfacing" of an antibody using selected germ-line amino acid residues for example, a predominantly germ-line framework in order to minimise the immunogenic effects of such an antibody when for example, used therapeutically. The process of the present invention is set out un claim 1.

The present invention provides processes for the production of antibodies with extremely low antigenicity. The antibodies may derive from an allogeneic antibody and have tracts of host-type predominantly germ-line V region framework sequences. Such processes would almost inevitably involve the replacement of exposed (surface) framework residues. Thus the processes of the present invention which may urilize germ-line V region frameworks for the antibody in question, provide for the easy and efficient creation of an antibody having a native host antibody surface.

Since it is not deemed acceptable to inject humans with an antigen of choice in order to harvest mature B cells, the majority of human monoclonal antibodies are created by techniques such as in vitro antigen priming of lymphocyte cultures or immunisation of hu-SCID mice.

In such techniques, relatively little, or no antibody maturation may take place prior to immortalisation via EBV transformation, or somatic cell fusion. This may result in the inadvertent production of antibodies with germ-line V region frameworks and also to a large degree, germ-line CDRs.

However, since little, or no, somatic mutation and antibody maturation will have taken place, and there is a limited size to the repertoire of V region genes, these antibodies will likely have low affinity for the antigen. Furthermore, there will have been no special selection for germ-line sequences. In particular, the sequence of the germ-line V regions would be unknown and could only be determined after the monoclonal producing cell line had been established.

In comparison, the processes of the present invention provide for the deliberate construction of altered antibodies with selected germ-line amino acid residues which replace one or more corresponding somatically mutated residues in a native antibody. The selected germ-line amino acid residues may comprise known germ-line V region framework sequences.

The processes of the present invention, provide for the grafting of mature, somatically mutated CDRs which impart high affinity binding to a germ-line V region framework. Such a construct could not be produced by priming and immortalization by hybridoma or alternative technologies. As discussed previously, even if there is some antibody maturation, thereby improving affinity, the somatic mutations would not be confined to the CDRs alone and mutations would also occur in the framework regions, so that their sequences would significantly deviate from that of the original germ-line. Thus where no maturation has occurred, the result would be a very low affinity antibody with germ-line framework regions. Alternatively, when maturation has occurred, the result would be a higher affinity antibody, but with framework regions deviating from germ-line sequences. Furthermore, the CDRs and V region frameworks will have come from the same species and very likely the same individual source.

In the processes of the present invention, the selected germ-line amino acid residues may derive from a different individual source/species than the source/species of the native antibody. For example, the CDR sequences and the germ-line V region framework sequences could, and in fact are likely to be derived from different individual sources and might also be derived from different species.

The present invention is applicable to antibodies especially for use in disease therapy or for in-vivo diagnosis. The successful targeting of antibody molecules to sites of disease upon repeated administration is dependent on these antibodies provoking little or no immune reaction. Maximisation of the germ-line V region content of these antibodies will minimise any immune reaction in response to their repeat administration. Therefore these antibodies will be of more use, for example, in the treatment of chronic disease.

It should also be noted that some mature antibody V region frameworks appear to possess biological acitivity such as protein A binding in the human VHIII gene family. Autoantibodies provide another example of activity, for instance, rheumatoid arthritis autoantibodies bind to the Fc of immunoglobulin molecules. The VK gene of a human polyarthritis autoantibody when compared to antibody of the VKIII family of normal individuals appears to differ in only residue 62 in framework 3 (phenylalanine to valine) from both the antibody GM60 and the germ-line sequence K562. The CDRs of these antibodies are identical. This suggests that even minor framework substitutions in the V region may have potent effects. Similarly V-region frameworks may also carry mimics of external antigens. For example, the common idiotype defined by 16.6 may be created inadvertantly by a single somatic mutation of particular frameworks. Therefore, unnecessary V region framework mutations may be deleterious and hence the use of germ-line V region frameworks and minimal sequence changes will be advantageous.

The processes of the present invention provide an altered antibody which comprises one or more selected germ-line amino acid residues which replace one or more corresponding somatically mutated residues in a native antibody. The altered antibody may conserve one or more amino acid residues which are essential to the antigen binding capacity of said native antibody.

The altered antibody may have variable (V) regions which comprise complementarity determining regions (CDRs) which provide the antibody which capacity to bind a specific antigen; and a selected and predominantly germ-line framework.

The CDRs may comprise one or more residues from germ-line CDR coding sequences; and one or more residues from an antibody V region gene which has undergone somatic mutation during B cell maturation. The framework may comprise one or more residues from an antibody V region gene which has undergone somatic mutation during B cell maturation, and which are essential to the antigen binding capacity of the antibody.

The selected germ-line framework may be homologous to the framework of an antibody V region gene which has undergone somatic mutation during B cell maturation. In which case, the antibody V region gene may comprise the CDRs.

The CDRs and selected germ-line framework may derive from different species.

The selected germ-line framework may form outer surfaces of the antibody.

The invention provides a process for making a gene for use in preparing an altered antibody which comprises: identifying one or more somatically mutated amino acid residues in a native antibody as suitable candidate(s) for alteration; and making a nucleotide coding sequence which codes for selected germ-line amino acid residues to replace said identified one or more somatically mutated amino acid residues.

The process may comprise making said nucleotide coding sequence so that it codes for one or more amino acid residues which are essential to the antigen binding capacity of the native antibody.

The invention also provides a process for making a gene for use in preparing an altered antibody as described above, which process has the following steps:
(1) obtaining CDR encoding nucleotide sequences which encode the CDRs; and (2) combining these CDR encoding nucleotide sequences with framework encoding nucleotide sequences which encode the selected germ-line framework.

The process may include the step of replacing one or more residues in the CDR encoding nucleotide sequences with corresponding different residues from germ-line CDR coding sequences.

The process may include the step of replacing one or more residues in said framework encoding nucleotide sequences with different residues from the framework of an antibody V region which has undergone somatic mutation during B cell maturation and wherein the different residues are essential to the antigen binding capacity of the antibody.

The process may comprise selecting the framework of the germ-line V region on the basis of homology to the framework of an antibody V region gene which has undergone somatic mutation during B cell maturation.

The process may comprise selecting the framework of the germ-line V region on the basis of homology to the framework of an antibody V region gene, which gene has undergone somatic mutation during B cell maturation and encodes said CDRs.

The CDR encoding nucleotide sequences may be grafted onto a gene for a germ-line V region. The framework encoding nucleotide sequences may replace nucleotide sequences coding for the framework of an antibody V region gene which has undergone somatic mutation during B cell maturation.

The gene may encode an antibody heavy chain, an antibody light chain, or fragments thereof.

The process may comprise the step of selecting CDR encoding and framework encoding nucleotide sequences which derive from different species.

The invention also provides a process which comprises expressing a gene obtainable by a process as described above.

The invention also provides a process which comprises formulating a pharmaceutical or diagnostic preparation having as a component, an antibody as described above.

Any germ-line variable region, including any germline equivalents of human frameworks (for example, the NEWM and/or KOL (VH) and REI (VK) variable region frameworks which have been successfully used in a "limited" or "fixed framework" strategy for antibody humanisation) may be used in the processes of the present invention. The "fixed framework" approach involves using a limited number of V region germ-line equivalent human frameworks onto which,
a) the CDRs of the antibody in question are grafted; and
b) introducing only those framework murine residues which are essential to retain the same level of antigen binding as the original antibody.

This strategy of using fixed frameworks for humanisation is well established and has been reported in the patent application no. PCT/GB91/01554 and Tempest P. et al. Biotechnology 9 p266-271 (1991).

Protein Design Labs in application WO 90/07861 and Queen et al. PNAS 86 (1989) p10029-10033 teach the creation of a humanised antibody based on a murine antibody having the desired specificity. Firstly, a human variable region is selected on the basis of maximum homology to the murine variable region. The murine CDRs are then used to replace the human CDRs in the selected human variable region. They also taught inclusion of several murine framework residues thought to interact with the murine CDRs. Thus the resultant altered antibody, retained the desired binding properties (as derived from the original murine antibody). However, there is a chance that the additional murine framework residues might contribute to the antibody being seen as foreign when administered to humans.

WO 90/07861 does not however teach the use of germline variable regions for matching, subsequent CDR grafting, and possible framework adjustment. Therefore, based on the teaching of the present application, a new approach is provided in which a germ-line variable region (for example, a human germ-line variable region) may be selected on the basis of substantial homology to the variable region of a gene which has undergone somatic mutation, for example, to the variable region of a murine antibody.

The "fixed framework" approach within the context of the present invention and as described above, differs from the strategy of WO 90/07861 in so much that it uses a limited number of germ-line equivalents of V region frameworks which are not the most homologous match to the murine variable region comprising the CDRs used for the replacement. Furthermore, there is incorporation of only those essential framework residues which are essential in order to retain the same level of antigen binding as the original antibody.

Thus since there is a need for altered antibodies with minimal immunogenicity for the diagnosis, prevention and treatment of infectious and other types of diseases, the combination of minimal framework and/or CDR changes, combined with the strategy of using germline equivalent V region frameworks (fixed frameworks or otherwise) provides a new and highly attractive approach.

In certain circumstances, a germ-line V region framework may be known to, or suspected of carrying an epitope which mimics an external antigen. In which case, it would be preferable to use a germ-line V region framework equivalent which is known not to carry such an epitope. For example, the "fixed framework" approach could be advantageously used. This in conjunction with minimal sequence changes, would then represent the best chance of producing an "immuno-silent" antibody with the desired binding characteristics.

The relative benefits of a utilizing "immuno-silent" antibody equivalents as described herein, can be tested and assessed, for example, by investigating in vivo responses to the antibody, clearance time etc. in various laboratory animal models such as rodents and primates, and comparing to equivalent test results for the original antibody, the standard V region grafted form (if applicable) and the germ-line equivalent. SCID mice and in vitro antibody culture systems could also be used to yield comparative data on whether a particular form of an antibody elicits an immune response. When, for example, humanised antibodies are to be tested, SCID-hu mice or in vitro human-lymphocyte cultures may be used as assay systems. Similarly, patient antisera may be tested for response during the course of a treatment. Similar systems, appropriately adjusted, might be used for other species. The use of animal models as proposed by LoBuglio A.F. et al., 1989 supra. may aid in the preclinical evaluation of monoclonal antibodies with regard to their relative V region immunogenicity.

A process of the present invention, for creating a same species germ-line equivalent to a given antibody, comprises the following basic scheme.
(i) The heavy and light chain V region nucleotide sequences of a given antibody are determined.
(ii) Heavy and light chain germ-line V region sequences of the same species of antibody are identified from a collection of such predetermined sequences.
(iii) The V region framework sequences of the given antibody are replaced by the corresponding germ-line V region framework sequences.
(iv) Optionally, individual residues within the CDR sequences of the given antibody are replaced, as far as possible, by individual residues from the corresponding germ-line CDRs.

In another process of the present invention, CDRs from a given antibody of one species, are grafted onto germ-line V regions of a different species of antibody. This method comprises the following basic scheme.
(i) The heavy and light chain V region nucleotide sequence of a given antibody of one species are determined.
(ii) The heavy and light chain germ-line V region sequences of a different species of antibody are identified from a collection of such predetermined sequences or selected from a collection of preconstructed recombinants (fixed framework approach) comprising such germ-line V regions.
(iii) Either the V region framework sequences of the given antibody from one species are replaced by the corresponding germ-line V region framework sequences of another species, or CDR sequences from the V regions of the given antibody of one species are grafted onto corresponding germ-line V regions of the different species of antibody.
(iv) Optionally, individual residues within CDR sequences of the given antibody of one species are replaced, as far as possible, by individual residues from corresponding germ-line CDRs of different species.

It will be recognised that for step (i) of the basic scheme in both processes, the V region sequences for the given antibody which is to be created in germline form, could be derived, for example, from a hybridoma (or myeloma), from a population of primary B cells or, alternatively, from combinatorial libraries of V region genes with specific antibodies selected by antigen binding.

Steps (ii) to (iv) from the basic scheme of both processes above, can be subject to additional manipulations in order to achieve, as required, as high affinity a germ-line antibody as possible, with as high a content of germ-line sequences as possible.

In step (ii), it is therefore desirable to select germ-line V regions which are as closely homologous as possible to the given antibody V region, and as a minimum, from the same species homology group (for example, the same subgroup as defined by Kabat, E.A. et al., Sequences of Proteins of Immunological Interest, US Dept of Health and Human Services, US Government Printing Office) or from the homology group of the germline species most closely matched to the given antibody V region. For both processes, it will be recognized that the choice of germ-line V region genes, will be influenced by the frequency of allelic variation of these genes in the population such that preferably germline V region genes with little or no variation in the population are selected.

In some cases, it may be possible (but less desirable) to use a consensus germ-line sequence (being a consensus of germ-line sequences in a homology group).

In step (iii), it would be desirable to retain any amino acid residue from the given antibody which is likely to improve the binding affinity of the final germ-line antibody. This might, for example, include V region framework residues retained from the original antibody before conversion to germ-line.

In step (iv), it is desirable to retain only key residues in the CDRs from the given antibody which are involved in binding to the antigen. In most cases, the majority of heavy chain CDR3 residues of the given antibody will be retained in the germ line V region heavy chain CDR3 whilst, in other CDRs, few amino acid residues may have to be retained in the germ-line antibody.

Although step (iv) from the basic scheme of both processes is optional, it may be advantageous if the CDRs were also germ-line. It is likely that the VH CDR3 of the test clone will be unique (no germ-line counterpart), but the other CDRs will have a few amino acid substitutions compared with the germ-line gene. The maintenance of key residues in CDRs 1 and 2 need not be onerous if combined with bacteriophage display technology (McCafferty, J. et al., Nature, 348, 552-554, 1990). Thus for example VH CDR3 of the antibody with the desired specificity can be transplanted into the selected germ-line V region gene as indicated above, and the construct tested for binding. If changes in CDR 1 and 2 were required, mixed oligonucleotides designed to make all the changes in each of the CDRs can be assembled in the V-gene by SOE-PCR (Ho, S.N. et al., Gene, 77, 51-59, 1989). The repertoire of sequences can then be cloned into the phage and a library created. The phages-antibodies of highest affinity can then be selected and sequenced, to determine the substitutions in CDR 1 and 2 necessary for binding. The sequence with the appropriate binding properties, yet a minimum of changes from the germ-line would then be selected.

Therefore it may be seen that additional modifications to the basic scheme can be implemented very easily using current technology. For example, using antibody phage display technology (McCafferty J. et al., 1990 supra.) many variants of each CDR from the given antibody including different numbers of substitutions with germ-line residues can be screened. Similarly, many variations of framework residues from the given antibody in the germ-line frameworks can also be screened. Indeed, it is convenient to synthesise mixtures of oligonucleotides to include many different variants of CDRs and, if desired, variants of frameworks and to assemble these oligonucleotides into V region genes by, for example, PCR mediated overlap extension (Ho, S.N. et al., Gene, 77, 51-59, 1989), to clone into vectors for phage display and to use affinity chromatography and/or other panning techniques to select high affinity antibody V regions. The selected high affinity clones would then be sequenced to identify the variant(s) comprising V regions with the smallest number of non germ-line residues as discussed above.

Those skilled in the art will understand that minor modifications to the selected germ-line amino acid sequences in the variable region CDRs or frameworks may be required in order to increase binding to antigen. It will also be understood that there will be variation between different antibodies in the extent to which parts of CDRs contribute to binding. This means that there will be differences in the capacity of different antibodies to include germ-line CDR sequences. It will also be appreciated that germ-line framework and germline CDR sequences will either be derived from the same or a different species to the CDR residues associated with antigen binding. In addition, the germ-line V regions may be associated with a variety of different constant regions either from the same or different species or source.

Germ-line V regions may be produced either with, or without, associated antibody constant region domains, or produced as genes or proteins fused to non-antibody fragments, proteins, various labels or other moieties.

It is a particular feature of this invention that antibodies for pharmaceutical application may be produced comprising human germ-line sequences in the heavy and light chain V region frameworks, human constant region, and non-human CDRs although where possible with human germ-line residues within the CDRs.

The processes of the present invention provide a number of different ways for creating antibodies with germ-line V regions. Firstly, cloned DNA fragments comprising selected germ-line V regions may be obtained in replicable vectors and the CDR sequences in these V region genes may be substituted by CDR sequences from other sources to produce specific antigen binding (in the antibody resulting from expression of the substituted antibody genes). Secondly, the V region frameworks of existing somatically mutated antibody genes may be modified by substitution of one or more somatically mutated residues to the germ-line equivalent.

As described above, the processes of the present invention may use a single, or restricted number of germ-line equivalents of V region frameworks for all constructions relating to a particular species, (for example, the use of NEWM and/or KOL VH and REI VK variable domains for humanisation purposes and their germ-line equivalents) or instead may use the best matched germ-line equivalent of V region framework for each individual antibody.

The invention is illustrated but not limited by the following examples and with reference to the figures in which:

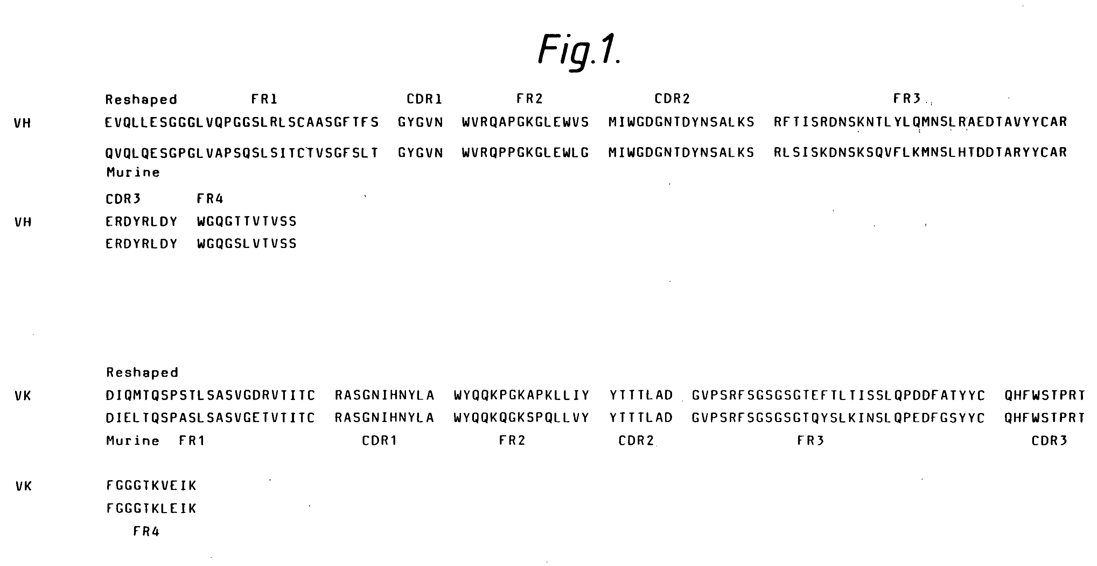

Figure 1 shows a comparison of the amino sequences of human germ-line reshaped (CDR-grafted) antibody ("Reshaped") Heavy (VH) and light (VK) chain variable domains in comparison to precursor murine antibody D1.3 ("Murine") sequences (Verhoeyen et al., 1988 supra). Frameworks ("FR 1-4") are derived from germ-line V and J genes H3HU26/JH6 and K1HU12/JK4 for VH and VK respectively. (H3HU26, K1HU12 are NBRF-PIR database ascension numbers).

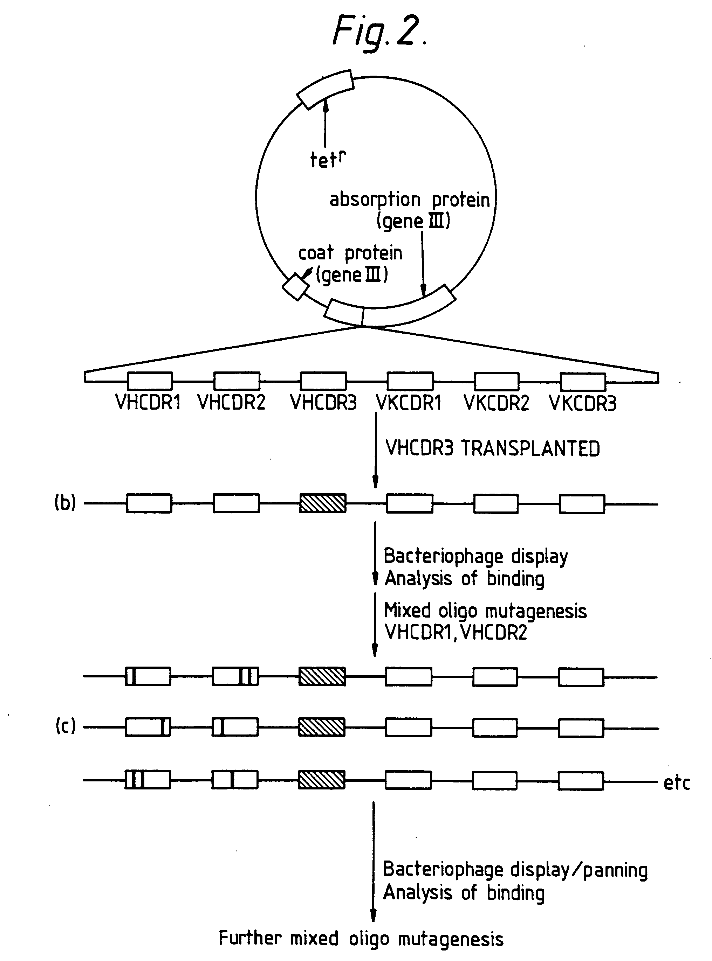

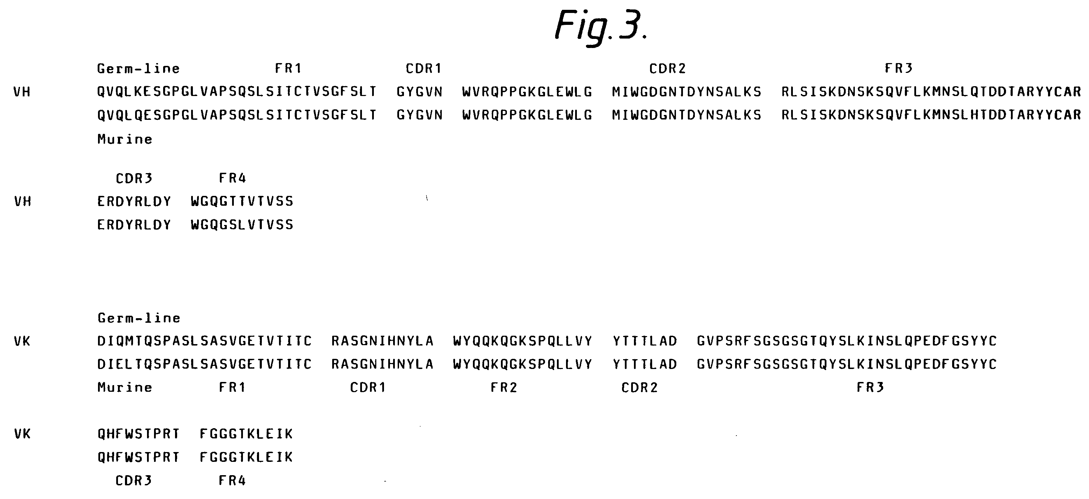

Figure 2 illustrates a methodology for minimising modification of germ-line CDRs in order to transfer only those CDR sequences necessary for antigen binding by the germ-line antibody. In stage (a), germ-line VH and VL domains would be assembled onto a suitable bacteriophage display vector such as Fd-CAT 1 (McCafferty et al., 1990 supra). In stage (b), the heavy chain CDR3 (VHCDR3) from the given monoclonal antibody for germ-line conversion would be substituted for the germ-line VHCDR3 and the resultant antibody variable domain tested for antigen binding either by phage display or by isolation of single-chain Fv fragments and analysis of binding. In stage (c), mixtures of oligonucleotides would then be used to effect the substitution of minimal numbers of germ-line VHCDR1 and VHCDR2 residues with corresponding residues from the given monoclonal antibody. Variable domains producing best binding affinities for antigen would then be selected via sequential rounds of bacteriophage growth and panning with antigen. If required, stage (c) may then be repeated to introduce further modifications either to CDRs or framework regions.
Figure 3 shows a comparison of a mouse germ-line converted mouse monoclonal antibody D1.3 (see Figure 1) with the original D1.3 VH and VK sequences. In this case, frameworks are derived from germ-line V and J genes HVMS14/JH2 and KVMSK2/JK1 from VH and VK respectively.

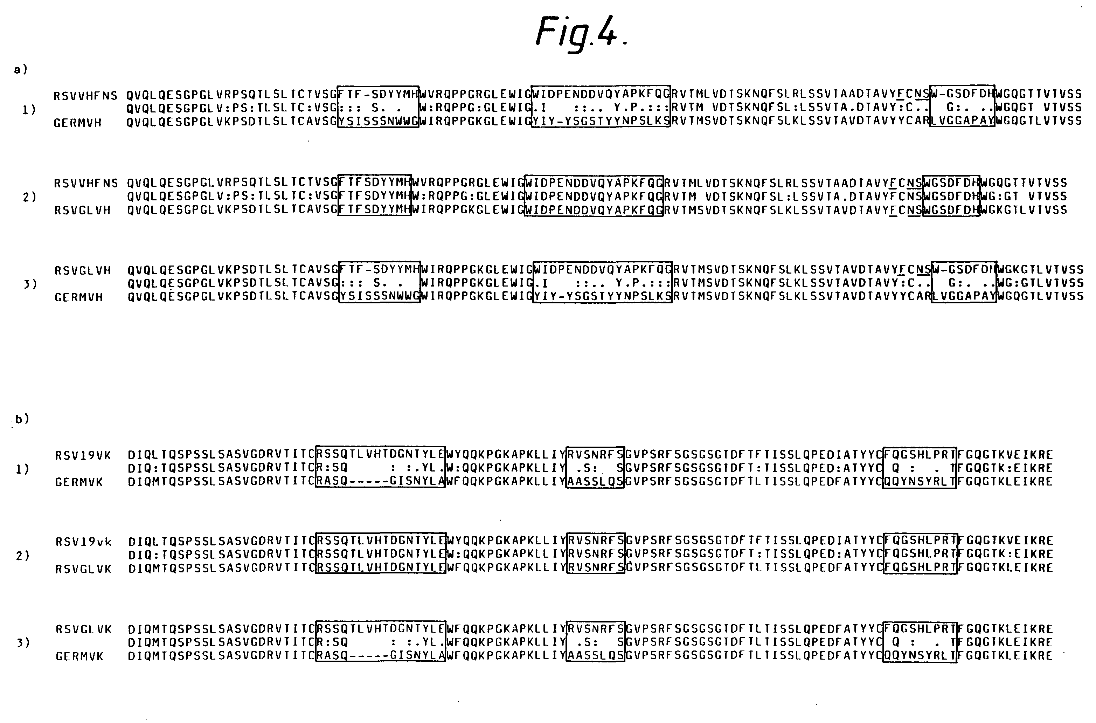

Figure 4 in sections a)1 and b)1 shows a comparison of matched human germ-lines with the original reshaped (humanised) equivalent HuRSV19FNS antibody (Tempest P. et al., 1991 supra). In this case, the germ-line frameworks were derived from the germ-line genes DP-68/JH6 (Tomlinson I. et al., 1992 supra) and HK137 for VH and VK respectively (GERMVH and GERMVK). In addition, comparisons of the germ-line converted RSV (RSVGLVH/VK) with the original humanised HuRSV19 antibody and the matched germ-line sequences are displayed in a) 2-3 and b) 2-3 to further illustrate the framework changes which have been made. Note: CDRs are boxed and essential framework residues underlined.

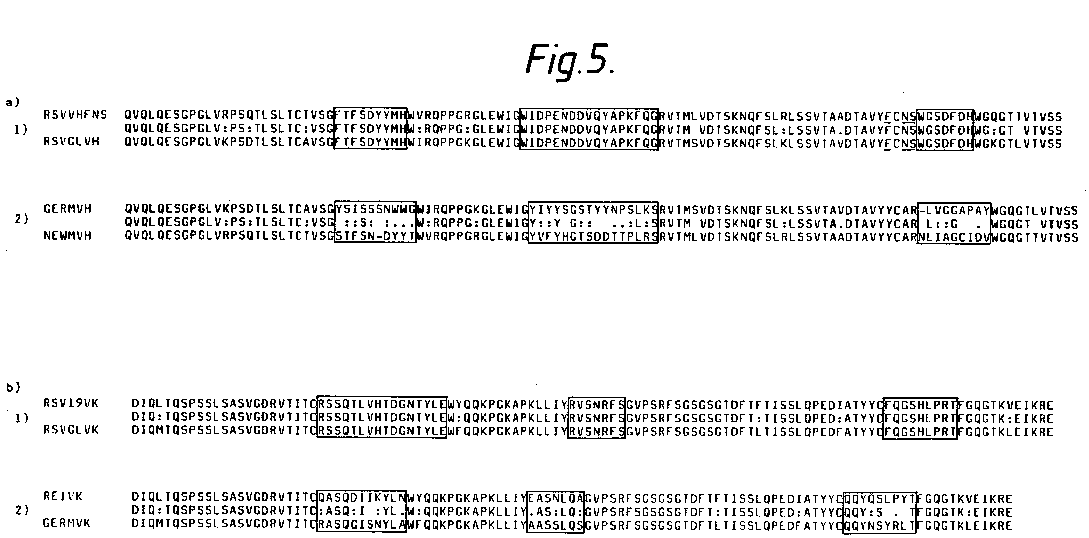

Figure 5 displays comparisons of the RSV germ-line antibody with a)1 and b)1 the original humanised RSV antibody. The comparisons in a)2 and b)2 are the original NEWM/REI antibody genes which were used as a basis for the original RSV humanisation aligned to the matched germ-line gene sequence information utilized in the production of the RSV germ-line equivalent. The figure illustrates that the germ-line sequence matches to the NEWM/REI framework and that amino acid substitutions made are within these framework regions. It may be seen from both this figure and figure 4 that although some CDR residues are common to the germ-line and the RSV or NEWM/REI antibodies the CDRs have diverged by a large degree to form the mature CDRs. Note: CDRs are boxed and essential framework residues underlined.

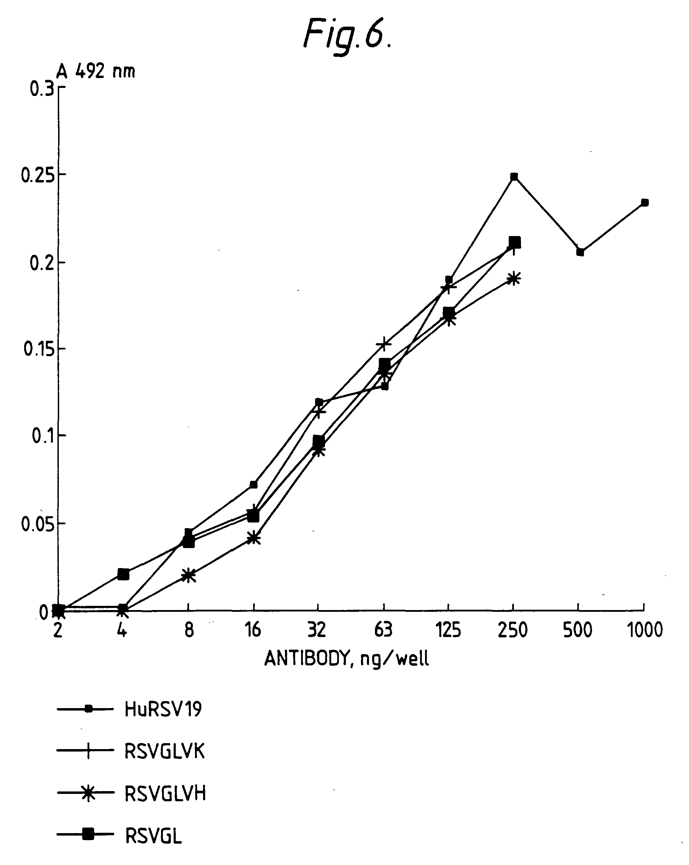

Figure 6 exhibits a graph representation of the results from the RSV19 germ-line ELISA. HuRSV19 is the original humanised RSV19FNS antibody, RSVGLVH the HuGLRSV19VHFNS/HuRSV19VK construct, RSVGLVK the HrRSV19HFNS/HuGLRSV19VK construct, and RSVGL the complete RSV19 germ-line antibody. The assay conditions and results are discussed within the text.

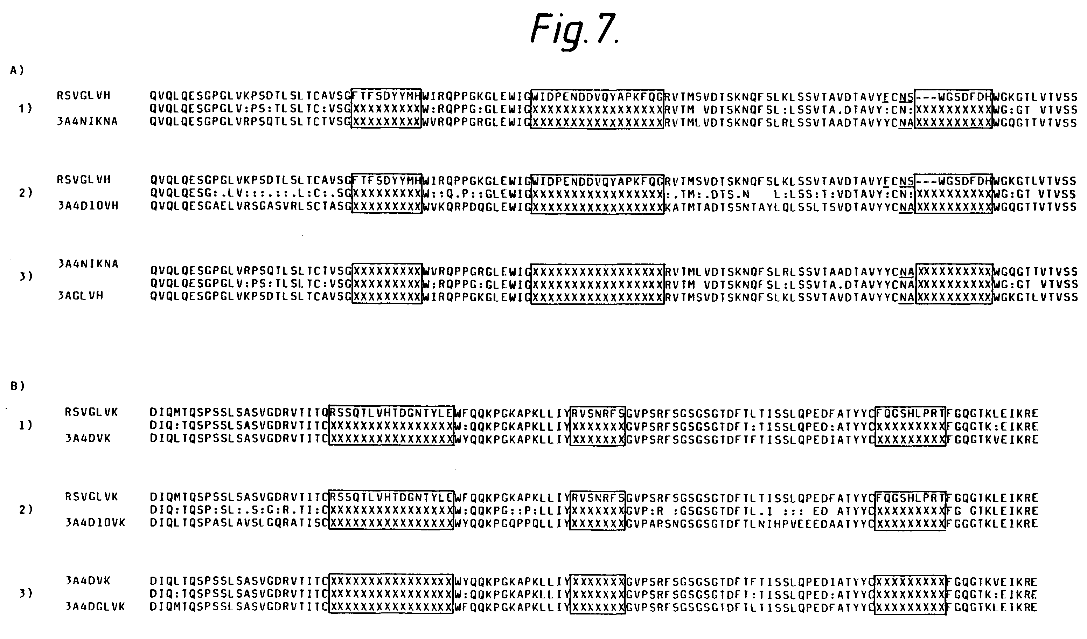

Figure 7 shows in sections a)1 and b)1 shows a comparison of the humanised 3a4D10 antibody with the RSV19 human germ-line antibody sequences. Sections a)2 and b)2 exhibit the mouse 3a4D10 antibody matched to the RSV19 human germ-line sequences. Sections a)3 and b)3 show a comparison of the humanised 3a4D10 antibody with its germ-line equivalent. The antigen affinity and specificity of the 3a4D10 antibody can be retained by grafting of the same CDR sequences (plus essential framework residues) as found in the mouse. This is true when either the conversion is made directly from a mouse antibody to a human germ-line framework antibody or from an equivalent humanised antibody to a corresponding human germ-line antibody. Note: All CDRs are boxed, the. 3a4D10 CDR residues are blanked out, and all essential framework residues are underlined.

### Example 1

This example illustrates the reshaping (humanisation) of a mouse monoclonal antibody by transfer of CDRs, to produce a corresponding humanised antibody with germ-line V region sequences.

Starting from a mouse hybridoma cell-line producing the monoclonal antibody of interest, a suitable method for determining the corresponding heavy and light chain variable sequences from RNA in the hybridoma is described by Orlandi et al., Proc. Natl. Acad. Sci. USA, 86, 3833-3837, 1989.

Genes encoding germ-line reshaped antibodies comprising mouse CDRs and human germ-line framework regions can be produced by site-directed mutagenesis to replace CDRs in germ-line heavy and light chain V regions (see Riechmann et al., 1988, supra). Alternatively, genes encoding germ-line reshaped antibodies can be assembled by gene synthesis (Jones P.T. et al., 1986, supra).

Figure 1 shows the amino acid sequences of a germline reshaped antibody comprising CDRs from the heavy and light chains of the mouse anti-lysozyme antibody D1.3 (Verhoeyen, M. et al., 1988, supra) with the corresponding germ-line frameworks region derived from H3HU26 (heavy chain) and K1HU12 (light chain). These germ-line framework regions are selected on the basis of close homology to the mouse D1.3 V region framework (shown in Figure 1 for comparison). Figure 1 also illustrates the inclusion in the germ-line framework of mouse D1.3 heavy chain amino acid residue 94 which is likely to promote efficient binding to lysozyme. Genes containing germ-line reshaped heavy and light chains can be cloned into replicable expression vectors and linked to genes for constant domains such as for human IgG1 (heavy chain) and human kappa (light chain) (see Orlandi et al., 1989, supra). These can then be cotransfected into mammalian cells for subsequent production of germline reshaped antibodies.

As an alternative to transfer of the complete CDR sequences from the V regions of a mouse monoclonal to germ-line V regions, methodology may be adopted for transfer of only CDR sequence components necessary for efficient antibody binding (Figure 2).

Thus, for example, genes encoding human germ-line heavy and light chains such as H3HU26 and K1HU12 respectively might be assembled into single-chain Fv's (scFv's) and the corresponding antibody V domains displayed on bacteriophage particles (McCafferty J. et al., 1990, supra). In the first instance, only the heavy chain CDR3 of the D1.3 antibody might then be transplanted into the selected heavy chain germ-line V region gene and the efficacy of lysozyme binding ascertained. Subsequently, if required, minimal substitutions of the germ-line heavy chain CDR1 and CDR2 might be implemented by mutagenesis at various positions using mixtures of oligonucleotides giving rise individually to alterations at these positions. By display on bacteriophage particles and by "panning" on a lysozyme affinity column, those mutant variable domains with the highest affinity for lysozyme could be selected. Similarly, minimally substituted germ-line light chain CDRs may be tested by bacteriophage particle display and panning.

An additional option for the conversion of monoclonal antibody to a corresponding germ-line antibody of another species may be accomplished by a two stage germ-line comparison of V region sequences followed by CDR modification. For example, comparing mouse V region sequences with mouse germ-line V region sequences. When good matches are achieved the appropriate mouse germ-line V sequences may be subsequently compared with human (or other species) germ-line sequences and the best match determined. This two step comparison approach may allow for the selection of a human (or other species) germ-line region which is highly suitable for further modification to produce the human germ-line equivalent of the mouse V region. The transfer of the complete or partial CDR sequences from the V regions of a mouse monoclonal to germ-line V regions in order to obtain the desired binding may then be performed. The comparisons made may, in addition to indicating which human germ-line V region framework is appropriate, also suggest which of the CDR component residues are necessary for efficient antibody binding.

### Example 2

This example illustrates the conversion of a given monoclonal antibody to its germ-line equivalent of the same species. Starting from cloned heavy and light chain V region domains corresponding to the given monoclonal antibody, conversion to germ-line is most conveniently achieved by site-directed mutagenesis of V region frameworks to convert these into germ-line. Alternatively, germ-line V regions may be produced by gene synthesis. Figure 3 shows germ-line derivates for heavy and light chain of the mouse monoclonal antibody D1.3 (see example 1) compared to the parent antibody sequence. In this example, germ-line V regions with good homology to the D1.3 heavy and light sequences (HVMS14 and KVMSK2 respectively) are selected as a basis for germ-line conversion. As described in the example, only key CDR residues from the D1.3 antibody might be transferred onto the matched germ-line V regions through display of V domains and panning of bacteriophage.

### Example 3

This example illustrates the conversion of a reshaped (CDR-grafted) antibody to its nearest germ-line equivalent. Figure 4 shows a comparison of the amino acid sequence of a human germ-line reshaped antibody with a pre-existing reshaped (humanised) antibody specific for respiratory Syncytial virus (Tempest P. et al., Biotechnology 9 p226-227, 1991). The reshaped antibody comprises CDRs originally from a mouse monoclonal antibody RSV19 specific for RSV, transplanted onto heavy and light chain V region domains derived from NEWM (Saul, F.A. et al., J. Biol. Chem. 253, 585-597, 1978) and REI (Epp, O. et al., Eur. J. Biochem. 45, 513-524, 1974) myeloma proteins respectively. In this example, conversion to germ-line variable regions (DP68/JH6 for VH and HK137 for VK) is most conveniently achieved by site-directed mutagenesis of the heavy and light chain reshaped antibody genes. Figure 4 also illustrates the inclusion in the germ-line framework of heavy chain amino acid residue 91 to 94 which derive originally from the RSV19 monoclonal antibody and which have been shown to be important for efficient binding to RSV (Tempest P. et al., Biotechnology 9 p226-271, 1991).

Again, if desired, only key CDR residues from the D1.3 antibody might be transferred onto the germ-line V regions and the desired antibody constructs selected through display of V domains and panning of bacteriophage as described in example 1.

### Experimental methods and results

The first of these trials involved the conversion of the humanised RSV19 antibody to a germ-line equivalent as proposed in example 3.

### Sequence Comparisons to obtain a matching Germ-line equivalents

The humanised RSV19 V region sequences (Tempest P. et al., Biotechnology 9 p226-271 1991) were compared to Germ-line sequences obtained either from Tomlinson et al., J. Mol. Biol. 227 p776-798, 1992, or data obtained from Genbank (Intelligenetics Inc) and comparisons were made using the DNASTAR program of Dnastar Inc. Figure 5 illustrates which residues were to be changed to convert the VH and VK variable domains to their equivalent germline sequences.

### Mutagenesis of the RSV19 VH and VK domains

It may be seen that the VK conversion involved the substitution of 5 amino acid residues while the VH required 10 such substitutions. In both cases, the amino acids to be substituted were dispersed fairly evenly throughout the sequences. The VH and VK Human RSV19 genes were cloned into M13 and single stranded DNA prepared from each construct. Five mutagenesis oligonucleotides were synthesised in order to convert the VK sequence, and another five synthesized in order to convert the VH sequence to the desired germ-line equivalent (Figure 6). The oligonucleotides were used simultaneously to effect the required DNA changes by oligonucleotide directed, site specific mutagenesis in a manner similar to Verhoeyen et al. Science, 239 p1534-1536, 1988. One pmole of each phosphorylated oligonucleotide was added to 500ng of single stranded DNA. Primers were annealed to the template by heating to 70°C and slowly cooling to room temperature. After site-directed mutagenesis, the DNA was transformed into competent E.coli TG1 cells. Single stranded DNA was prepared from the individual plaques and sequenced using the dideoxy method using Sequenase Trade Mack (United States Biochemicals). If only partial mutants were obtained, these were then subjected to further rounds of mutagenesis, using the appropriate oligonucleotides until the complete mutants were obtained. The germ-line RSV19 VH and VK genes were cloned into expression vectors (Orlandi et al., 1989 supra) to yield the plasmids termed pHuGLRSV19VHFNS and pHuGLRSV19VK. The GLVH gene together with the IgG heavy chain promoter, appropriate splice sites and signal peptide sequences was excised from M13 by digestion with HindIII and BamHI, and cloned into an expression vector containing the murine Ig heavy chain enhancer, a human IgG1 constant region, the SV40 promoter, the gpt gene for selection in mammalian cells and genes for replication and selection in E.coli. The construction of the pHuGLRSV19VK plasmid was essentially the same, except that in this construct the gpt gene was replaced by the hygromycin resistance gene and the IgG1 by the human kappa constant region.

### Antibody expression

5ug of either pHuGLRSV19VHFNS or pHuRSV19VHFNS and 10ug of either pHuGLRSV19VK or pHuRSV19VK were linearised with Pvul for the RSV19 antibody combinations GLVH/VK, VH/GLVK, GLVH/GLVK and the original VH/VK. The DNAs were mixed together, ethanol precipitated and dissolved in 25ul water. Approximately 10⁷ YB2/0 cells were grown to semiconfluency, harvested by centrifugation and resuspended in DMEM together with the digested DNA in an electroporation cuvette. After 5 minutes on ice, the cells were given a single pulse of 170v at 960 uF (Gene pulser, Bio-Rad) and left on ice for a further 20 minutes. The cells were then put into 20mls DMEM plus 10% FCS and allowed to recover for 48 hours. At this time the cells were distributed into a 24 well plate and selective medium applied (DMEM, 10% FCS, 0.8mg/ml mycophenolic acid, 250mg/ml Xanthine). After 3-4 days, the medium and dead cells were removed and replaced with fresh selective medium. Transfectant clones were visable to the naked eye 8-10 days later.

The antibody producing clones were tested by ELISA and the clones expanded until they were growing in a 600ml volume (in 5 flasks). Antibody was harvested using protein A precipitation and exclusion of antibody achieved by packing a small cartridge with the protein A-antibody material, washing with 0.1M Tris pH8.0, then 0.01M Tris pH8.0 and eluted using a 100mM glycine pH3.0 buffer. 1ml fractions were neutralised with 100ul 1M Tris pH8.0 and the OD/280 measured. The antibody containing fractions were pooled and the antibody dialysed against PBS buffer overnight. The antibodies were subsequently filter sterilized, the OD/280 measured and stored at +4°C.

The antigen binding assay to compare whether or not the "mix and match HgGLRSV/HuRSV and the complete HuGLRSV19 antibody binds as well as the original HuRSV19 was performed as described by Tempest P. et al., Biotechnology 9 p226-271, 1991.

The results of the various construct combinations are shown in Figure 6.

### Results

When assayed the germ-line HuRSV19 antibody construct exhibited the same binding properties as the original HuRSV19 antibody. Similarly the HuGLRSV19VHFNS/HuRSV19VK and HuRSV19VHFNS/HuGLRSV19VK constructs also showed no reduction in binding properties. These results thus indicated that the germline HuRSV19 heavy and light chains retained their individual antigen binding properties and that the complete human germ-line antibody exhibits the same binding characteristics as the HuRSV19 antibody from which it was derived. Thus, the framework substitutions made to convert the HuRSV19 antibody to its germ-line equivalent appear to have no detrimental effect on the binding properties of the antibody.

The second set of experiments involves the grafting of CDRs (and any essential framework residues) onto a germ-line variable region. In this experiment the antibody 3a4D10 (anti-Clostridium perfingens alpha toxin) which exists as mouse monoclonal and also as humanised antibody. The sequence of the CDRs are known and in addition the framework modifications essential for maintaining binding have been determined.

The V region sequences of the mouse and human 3a410 antibody (with the CDR blanked out) and their comparisons with the germ-line framework antibody are shown in figure 7. In this experiment the NEWM/REI germ-line V region framework (produced in the process of converting the existing RSV19 antibody to its germ-line equivalent) will be used as a base onto which the appropriate CDRs for the 3a4D10 will be grafted. The CDRs are identical with the original mouse antibody. Thus the experiment is designed to substantiate example 1 in which the CDRs of a monoclonal are transferred to a germ-line framework of a different species.

Since the antibody also exists in humanised form the experiment may also be seen to substantiate example 2 where the CDRs of an antibody are transferred to a germ-line framework of the same species. This approach differs from example 3 in such that a germ-line equivalent is being produced by the grafting of CDRs onto a framework and thus replacing the frameworks original CDRs. In example 3, the germ-line equivalent is being produced by conversion of the existing framework and therefore does not involve CDR grafting.

In all both cases the antibody's essential framework modifications must be made to retain binding and so must also be incorporated when constructing the germ-line antibodies.

The DNA manipulation and expression techniques involved in creating these constructs are identical to those discussed in the first experiment. To graft the six 3a4D10 CDRs onto the human germ-line NEWM/REI framework and to make the essential framework changes six oligonucleotides are required.

Micro-titre plate wells were coated overnight at 4°C with 1 microgram Phospholipase C type XiV (Sigma P4039). After blocking and washing the murine and test antibodies were applied for 1 hour at room temperature. The wells were emptied, washed and the immobilised antibodies were detected by incubation for 1 hour at room temperature with peroxidase conjugated goat antimouse or goat anti-human IgG antibodies (Sera-labs) diluted to 1:1000. The substrate for peroxidase activity was O-phenylenediamine (OPD) plus hydrogen peroxidase under standard buffer conditions.

## Claims

1. A process for making a gene for use in preparing an altered antibody, wherein one or more somatically mutated amino acid residues in a native antibody are replaced by corresponding germ-line amino acid residues at selected positions in a native antibody so that said altered antibody has immunogenicity lower than said native antibody, the process comprising:
identifying one or more somatically mutated amino acid residues in a native antibody as suitable candidate(s) for alteration; and
making a nucleotide coding sequence which codes for selected germ-line amino acid residues to replace said identified one or more somatically mutated amino acid residues.

2. The process of claim 1 which comprises making said nucleotide coding sequence such that it codes for one or more amino acid residues which are essential to the antigen binding capacity of said native antibody.

3. The process of claim 1 or claim 2 which conserves one or more amino acid residues which are essential to the antigen binding capacity of said native antibody.

4. The process of any one of claims 1 to 3, wherein the altered antibody has variable (V) regions which comprise:
complementarity determining regions (CDRs) which provide the antibody with capacity to bind a specific antigen; and
a selected and predominantly germ-line framework.

5. The process of claim 4, wherein the CDRs comprise:
one or more residues from germ-line CDR coding sequences; and
one or more residues from an antibody V region gene which has undergone somatic mutation during B cell maturation.

6. The process of claim 4 or claim 5, wherein the framework comprises one or more residues from an antibody V region gene which has undergone somatic mutation during B cell maturation, and which are essential to the antigen binding capacity of said antibody.

7. The process of any one of claims 4 to 6, wherein the selected germ-line framework is homologous to the framework of an antibody V region gene which has undergone somatic mutation during B cell maturation.

8. The process of claim 7, wherein said antibody V region gene comprise said CDRs.

9. The process of any one of claims 4 to 8, wherein the CDRs and selected germ-line framework derive from different species.

10. The process of any one of claims 4 to 9, wherein said selected germ-line framework forms outer surfaces of said antibody.

11. The process of claim 4 wherein the process comprises:
(1) obtaining CDR encoding nucleotide sequences which encode said CDRs; and
(2) combining these CDR encoding nucleotide sequences with framework encoding nucleotide sequences which encode said selected germ-line framework.

12. The process of claim 11 which includes the step of
replacing one or more residues in the CDR encoding nucleotide sequences with corresponding different residues from germ-line CDR coding sequences.

13. The process of claim 11 or claim 12 which includes the step of:
replacing one or more residues in said framework encoding nucleotide sequences with different residues from the framework of an antibody V region which has undergone somatic mutation during B cell maturation and
wherein said different residues are essential to the antigen binding capacity of said antibody.

14. The process of any one of claims 11 to 13 which comprises selecting the framework of the germ-line V region on the basis of homology to the framework of an antibody V region gene which has undergone somatic mutation during B cell maturation.

15. The process of claim 14 which comprises selecting the framework of the germ-line V region on the basis of homology to the framework of an antibody V region gene which gene has undergone somatic mutation during B cell maturation and which encodes said CDRs.

16. The process of any one of claims 12 to 15, wherein said CDR encoding nucleotide sequences are grafted onto a gene for a germ-line V region.

17. The process of any one of claims 12 to 16, wherein said framework encoding nucleotide sequences replace nucleotide sequences coding for the framework of an antibody V region gene which has undergone somatic mutation during B cell maturation.

18. The process of any one of claims 12 to 16, wherein the gene encodes an antibody heavy chain or fragment thereof.

19. The process of any one of claims 12 to 16, wherein the gene encodes an antibody light chain or fragment thereof.

20. The process of any one of claims 12 to 19 which comprises the step of selecting CDR encoding and framework encoding nucleotide sequences which derive from different species.

21. A process for making an antibody which comprises expressing a gene obtainable by a process according to any one of claims 1 to 20.

22. The process of claim 21, further comprising the step of formulating the antibody as a diagnostic or therapeutic composition.

## Patentansprüche

1. Verfahren zur Herstellung eines Gens zur Erzeugung eines veränderten Antikörpers, worin ein oder mehrere somatisch mutierte Aminosäurereste in einem nativen Antikörper an ausgewählten Positionen in einem nativen Antikörper durch entsprechende Keimlinien-Aminosäurereste ersetzt werden, so dass der veränderte Antikörper geringere Immunogenität aufweist als der native Antikörper, wobei das Verfahren Folgendes umfasst:
das Identifizieren eines somatisch mutierten Aminosäurerests oder mehrerer somatisch mutierter Aminosäurereste in einem nativen Antikörper als geeigneter Kandidat bzw. geeignete Kandidaten für die Veränderung; und
das Herstellen einer Nukleotid-Kodierungssequenz, die für die ausgewählten Keimlinien-Aminosäurereste kodiert, um den einen oder die mehreren identifizierten somatisch mutierten Aminosäurerest(e) zu ersetzen.

2. Verfahren nach Anspruch 1, welches das Herstellen der Nukleotid-Kodierungssequenz auf solche Weise umfasst, dass sie für einen oder mehrere für die Antigenbindungsfähigkeit des nativen Antikörpers essentielle(n) Aminosäurerest(e) kodiert.

3. Verfahren nach Anspruch 1 oder 2, bei dem ein oder mehrere für die Antigenbindungsfähigkeit des nativen Antikörpers essentielle(r) Aminosäurerest(e) konserviert wird bzw. werden.

4. Verfahren nach einem der Ansprüche 1 bis 3, worin der veränderte Antikörper variable (V) Regionen aufweist, die Folgendes umfassen:
Komplementarität bestimmende Regionen (CDRs), die dem Antikörper die Fähigkeit verleihen, ein spezifisches Antigen zu binden; sowie
einen ausgewählten und vorwiegend von Keimlinien herrührenden Rahmen.

5. Verfahren nach Anspruch 4, worin die CDRs Folgendes umfassen:
einen oder mehrere Reste von Keimlinien-CDR-Kodierungssequenzen; und
einen oder mehrere Reste von einem Antikörper-V-Region-Gen, das während der B-Zellen-Reifung somatische Mutation erfahren hat.

6. Verfahren nach Anspruch 4 oder 5, worin der Rahmen einen oder mehrere für die Antigen-Bindungsfähigkeit des Antikörpers essentielle(n) Rest(e) von einem Antikörper-V-Region-Gen umfasst, das während der B-Zellen-Reifung somatische Mutation erfahren hat.

7. Verfahren nach einem der Ansprüche 4 bis 6, worin der ausgewählte Keimlinien-Rahmen zum Rahmen eines Antikörper-V-Region-Gens homolog ist, das während der B-Zellen-Reifung somatische Mutation erfahren hat.

8. Verfahren nach Anspruch 7, worin das Antikörper-V-Region-Gen die CDRs umfasst.

9. Verfahren nach einem der Ansprüche 4 bis 8, worin die CDRs und der ausgewählte Keimlinien-Rahmen von unterschiedlichen Spezies stammen.

10. Verfahren nach einem der Ansprüche 4 bis 9, worin der ausgewählte Keimlinien-Rahmen äußere Oberflächen des Antikörpers bildet.

11. Verfahren nach Anspruch 4, worin das Verfahren Folgendes umfasst:
(1) das Erhalten von CDR-kodierenden Nukleotidsequenzen, die für die CDRs kodieren; und
(2) das Kombinieren dieser CDR-kodierenden Nukleotidsequenzen mit Rahmen-kodierenden Nukleotidsequenzen, die für den ausgewählten Keimlinien-Rahmen kodieren.

12. Verfahren nach Anspruch 11, welches des Schritt
des Ersetzens eines oder mehrerer Reste in den CDR-kodierenden Nukleotidsequenzen durch entsprechende andere Reste von Keimlinien-CDR-Kodierungssequenzen umfasst.

13. Verfahren nach Anspruch 11 oder 12, welches den Schritt
des Ersetzens eines oder mehrerer Reste in den Rahmen-kodierenden Nukleotidsequenzen durch andere Reste aus dem Rahmen einer Antikörper-V-Region umfasst, die während der B-Zellen-Reifung somatische Mutation erfahren hat, und
worin die anderen Reste für die Antigenbindungsfähigkeit des Antikörpers essentiell sind.

14. Verfahren nach einem der Ansprüche 11 bis 13, welches die Selektion des Rahmens der Keimlinien-V-Region auf der Basis von Homologie mit dem Rahmen eines Antikörper-V-Region-Gens umfasst, das während der B-Zellen-Reifung somatische Mutation erfahren hat.

15. Verfahren nach Anspruch 14, welches die Selektion des Rahmens der Keimlinien-V-Region auf der Basis von Homologie mit dem Rahmen eines Antikörper-V-Region-Gens umfasst, wobei das Gen während der B-Zellen-Reifung somatische Mutation erfahren hat und für die CDRs kodiert.

16. Verfahren nach einem der Ansprüche 12 bis 15, worin die CDR-kodierenden Nukleotidsequenzen auf ein Gen für eine Keimlinien-V-Region verpflanzt werden.

17. Verfahren nach einem der Ansprüche 12 bis 16, worin die Rahmen-kodierenden Nukleotidsequenzen Nukleotidsequenzen ersetzen, die für den Rahmen eines Antikörper-V-Region-Gens kodieren, das während der B-Zellen-Reifung somatische Mutation erfahren hat.

18. Verfahren nach einem der Ansprüche 12 bis 16, worin das Gen für eine Antikorper-Schwerkette oder ein Fragment davon kodiert.

19. Verfahren nach einem der Ansprüche 12 bis 16, worin das Gen für eine Antikörper-Leichtkette oder ein Fragment davon kodiert.

20. Verfahren nach einem der Ansprüche 12 bis 19, welches den Schritt der Selektion von CDR-kodierenden und Rahmen-kodierenden Nukleotidsequenzen umfasst, die von unterschiedlichen Spezies stammen.

21. Verfahren zur Herstellung eines Antikörpers, welches das Exprimieren eines Gens umfasst, das nach einem Verfahren nach einem der Ansprüche 1 bis 20 erhältlich ist.

22. Verfahren nach Anspruch 21, das weiters den Schritt des Formulierens des Antikörpers als Diagnose- oder Therapie-Zusammensetzung umfasst.

## Revendications

1. Procédé de fabrication d'un gène à utiliser dans la préparation d'un anticorps modifié, où un ou plusieurs résidus d'acides aminés somatiquement mutés dans un anticorps natif sont remplacés par des résidus d'acides aminés de lignée germinale correspondante en des positions sélectionnées dans un anticorps natif de façon que ledit anticorps modifié ait une immunogénicité plus faible que ledit anticorps natif, ledit procédé comprenant:
l'identification d'un ou plusieurs résidus d'acides aminés somatiquement mutés dans un anticorps natif comme un ou des candidats appropriés pour la modification; et
la fabrication d'une séquence de codage de nucléotides qui code pour des résidus d'acides aminés de lignée germinale sélectionnée pour remplacer le ou les résidus d'acides aminés somatiquement mutés identifiés.

2. Procédé de la revendication 1 qui consiste à faire ladite séquence de codage de nucléotides telle qu'elle code pour un ou plusieurs résidus d'acides aminés qui sont essentiels pour la capacité de liaison d'antigène dudit anticorps natif.

3. Procédé de la revendication 1 ou de la revendication 2 qui concerne un ou plusieurs résidus d'acides aminés qui sont essentiels pour la capacité de liaison de l'antigène dudit anticorps natif.

4. Procédé de l'une quelconque des revendications 1 à 3, où l'anticorps modifié a des régions variables (V) qui comprennent:
des régions déterminant la complémentarité (CDR) qui donnent à l'anticorps la capacité de lier un antigène spécifique; et
une charpente sélectionnée et de manière prédominante de lignée germinale.

5. Procédé de la revendication 4, où les CDR comprennent:
un ou plusieurs résidus de séquences de codage de CDR de lignée germinale; et
un ou plusieurs résidus d'un gène d'une région V d'un anticorps qui a subi une mutation somatique pendant la maturation des cellules B.

6. Procédé de la revendication 4 ou de la revendication 5, où la charpente comprend un ou plusieurs résidus d'un gène d'une région V d'anticorps qui a subi une mutation somatique pendant la maturation des cellules B et qui sont essentiels pour la capacité de liaison de l'antigène dudit anticorps.

7. Procédé selon l'une quelconque des revendications 4 à 6, où la charpente de lignée germinale sélectionnée est homologue avec la charpente d'un gène de région V d'anticorps qui a subi une mutation somatique pendant la maturation des cellules B.

8. Procédé de la revendication 7, où ledit gène de la région V de l'anticorps comprend lesdites CDR.

9. Procédé selon l'une quelconque des revendications 4 à 8, où les CDR et la charpente de lignée germinale sélectionnée dérivent d'espèces différentes.

10. Procédé selon l'une quelconque des revendications 4 à 9, où ladite charpente de lignée germinale sélectionnée forme les surfaces externes dudit anticorps.

11. Procédé de la revendication 4 où le procédé comprend:
(1) l'obtention de séquences de nucléotides codant pour des CDR qui codent pour lesdites CDR; et
(2) la combinaison de ces séquences de nucléotides codant pour des CDR avec des séquences de nucléotides codant pour une charpente qui codent pour ladite charpente de lignée germinale sélectionnée.

12. Procédé de la revendication 11 qui comprend l'étape de
remplacer un ou plusieurs résidus dans les séquences de nucléotides codant pour les CDR par des résidus différents correspondants de séquences codant pour les CDR de lignée germinale.

13. Procédé de la revendication 11 ou de la revendication 12 qui comprend l'étape de :
remplacer un ou plusieurs résidus dans lesdites séquences de nucléotides codant pour la charpente par des résidus différents de la charpente d'une région V d'anticorps qui a subi une mutation somatique pendant la maturation des cellules B et
où lesdits résidus différents sont essentiels pour la capacité de liaison de l'antigène dùdit anticorps.

14. Procédé selon l'une quelconque des revendications 11 à 13 qui comprend la sélection de la charpente de la région de lignée germinale V sur la base de l'homologie à la charpente d'un gène de région d'anticorps V qui a subi une mutation somatique pendant la maturation des cellules B.

15. Procédé de la revendication 14 qui comprend la sélection de la charpente de la région de lignée germinale V sur la base de l'homologie avec la charpente d'un gène de région d'anticorps V, lequel gène a subi une mutation somatique pendant la maturation des cellules B et qui code pour lesdites CDR.

16. Procédé selon l'une quelconque des revendications 12 à 15 où lesdites séquences de nucléotides codant pour les CDR sont greffées sur un gène pour une région de lignée germinale V.

17. Procédé de l'une quelconque des revendications 12 à 16, où lesdites séquences de nucléotides codant pour la charpente remplacent les séquences de nucléotides codant pour la charpente d'un gène de région d'anticorps V qui a subi une mutation somatique pendant la maturation des cellules B.

18. Procédé de l'une quelconque des revendications 12 à 16, où le gène code pour une chaîne lourde d'anticorps ou son fragment.

19. Procédé de l'une quelconque des revendications 12 à 16, où le gène code pour une chaîne légère d'anticorps ou son fragment.

20. Procédé selon l'une quelconque des revendications 12 à 19 qui comprend l'étape de sélectionner des séquences de nucléotides codant pour les CDR et codant pour la charpente qui dérivent d'espèces différentes.

21. Procédé pour faire un anticorps qui comprend l'expression d'un gène pouvant être obtenu par un procédé selon l'une quelconque des revendications 1 à 20.

22. Procédé de la revendication 21, comprenant de plus l'étape de formuler l'anticorps en tant que composition de diagnostic ou thérapeutique.
